Europäisches Patentamt

(19) European Patent Office

Office européen des brevets

(11) Publication number: **0 097 436**
**B1**

(12) **EUROPEAN PATENT SPECIFICATION**

(45) Date of publication of patent specification: **12.08.87**

(21) Application number: **83303052.1**

(22) Date of filing: **26.05.83**

(51) Int. Cl.⁴: **A 61 N 1/04, A 61 N 1/30**

(54) Bioelectrode.

(30) Priority: **16.06.82 US 388987**

(43) Date of publication of application:
**04.01.84 Bulletin 84/01**

(45) Publication of the grant of the patent:
**12.08.87 Bulletin 87/33**

(84) Designated Contracting States:
**DE FR GB IT SE**

(56) References cited:
**EP-A-0 029 297**
**EP-A-0 060 451**
**DE-A-3 006 472**
**DE-A-3 215 960**
**FR-A-2 291 732**
**GB-A- 410 009**
**GB-A-2 045 088**
**US-A-3 976 055**
**US-A-4 248 247**

**The file contains technical information
submitted after the application was filed and
not included in this specification**

(73) Proprietor: **MINNESOTA MINING AND
MANUFACTURING COMPANY
3M Center, P.O. Box 33427
St. Paul, Minnesota 55133-3427 (US)**

(72) Inventor: **Roberts, Charles W. Minnesota Mining
and Manufact.
Company 2501 Hudson Road P.O. Box 33427
Saint Paul Minnesota 55133 (US)**

(74) Representative: **Baillie, Iain Cameron et al
c/o Ladas & Parry Isartorplatz 5
D-8000 München 2 (DE)**

Courier Press, Leamington Spa, England.

## Description

This invention relates to biomedical electrodes, and in particular, to biomedical electrodes used to deliver current to the body as in, for example, transcutaneous electrical nerve stimulation (TENS), functional electrical stimulation (F.E.S.) and iontophoretic drug delivery.

Two primary concerns which must be addressed in the design of bioelectrodes for delivering current to the body are patient comfort and safety. To maximize comfort and safety, stimulating bioelectrodes, e.g., TENS electrodes, are generally constructed of flexible materials such as conductive mesh (U.S. Patent No. 4,243,051), metal foil (U.S. Patent No. 4,125,110), or silicone rubber (published United Kingdom application No. 2,045,088) which conform to the contours of the body over which they are adhered. If good contact is maintained between the electrode and the skin, and if the electrode delivers uniform current densities over its entire surface, the potential for development of "hot spots". and their attendant discomfort and possible burning, is minimized.

While the prior art has been moderately successful in providing biomedical electrodes which exhibit good conformability to skin, the aforementioned types of electrodes all suffer from certain disadvantages.

Biomedical electrodes such as those described in published United Kingdom application No. 2,045,088, made of conductive silicone rubber, are rather bulky and heavy, comparatively speaking, and less supple than electrodes formed from thinner materials such as conductive mesh or foil. They also tend to be more expensive and more power consuming than the other types of electrodes. While their disadvantages are not of great significance in the case of relatively small TENS electrodes used to treat chronic pain such as lower back pain, they are magnified in the case of larger electrodes such as those used in the management of post-operative pain. In this application, one electrode, often as long as 25 cms (ten inches), is placed on each side of the incision, and therefore thin, supple electrodes are particularly desirable.

US-A-4 248 247 discloses an electrode consisting of a non-metallic flexible conductive strip preferably formed from a synthetic material such as rubber which is impregnated with electrically-conducted particles such as carbon. The conductive layer is carried by a non-conductive backing layer which also serves to trap an electrical connecting wire between itself and the conductive layer providing electrical contact thereto. This electrode is defined in the first part of claim 1.

Electrodes formed of a conductive mesh such as the aluminium-coated fabric described in U.S. Patent No. 4,243,051 or a metal foil can be made thin, light-weight and supple, particularly when a relatively thin layer of a conductive adhesive is used as the interfacing material between the electrode and the skin to reduce impedance and adhere the elctrode to the skin. However, a major problem has been discovered recently in connection with metal electrodes used to deliver current to the body. This problem involves the migration of metal ions from the electrode to the skin under the influence of direct current produced by many stimulator units now in use. Stimulators used in TENS therapy are designed to provide a carefully patterned stream of electrical impulses with no D.C. component. It has been discovered, however, that certain stimulators currently in use may produce a significant D.C. component which is capable of electrolyzing metal in the electrode. In electrodes with an aqueous pathway between the electrode and the skin, such as that provided by a typical electrolyte gel or a conductive adhesive dependent on moisture or humidity, deplating of the electrode occurs. A vapor-coated silver layer, for example, can be deplated by one of these stimulators in a few hours. Since the electrodes are often designed to remain on the body for several days, the silver is deposited in the skin, and the effective contact area for current conduction is localized under the point of termination of the lead wire producing a "hot spot" which can result in extreme discomfort and even "burning".

The bioelectrode of the present invention effectively prevents the aforementioned problem of deplating and migration of metallic ions while maintaining all of the desirable features of metal electrodes such as thinness, light-weight, suppleness, low cost, etc.

According to the present invention there is provided a biomedical electrode comprising an electrically-conductive metallic member, means for electrically connecting said metallic member to an electromedical device, a flexible, electrically-conductive, non-metallic film which is a barrier to the migration of metallic ions, said non-metallic film having a top surface and a bottom surface, and an extensible, electrically insulating backing overlies and is adhered to said metallic member on the side opposite to the side contacting said film; characterised in that said electrically-conductive metallic member is in the form of a layer which overlies and is in electrical contact with substantially all of said top surface of said film, thereby conducting evenly over the entire area of the elctrode. Optional features of the invention are set out in claims 2-16 below.

Preferably, the bioelectrode of the invention is disposable and further comprises a conformable, electrically-conductive interfacing material on the bottom surface of the electrically-conductive film. In the preferred embodiment, the interfacing material is a layer of a conductive adhesive which is sufficiently tacky to adhere the entire electrode to the skin. In the preferred embodiment, a protective release liner is adhered to the conductive adhesive to protect the adhesive prior to use.

The bioelectrode of the invention is designed to provide uniform current densities over the entire surface in contact with the skin. It is relatively thin, supple, light-weight and not affected by direct current which may be generated by stimulators currently in use.

The term "flexible" and "conformable" are used interchangeably herein to refer to the ability to conform to the contours of the body over which the bioelectrode is placed to provide a high surface area of contact between the skin and the elctrode.

The term "extensible" as used herein in connection with the backing portion of the bioelectrode refers to the ability of the backing to stretch when mechanical force is applied to it so as not to exert sheer forces on the metal layer to which it is adhered and thereby cause the metal layer to delaminate from and lose electrical contact with the underlying electrically-conductive film.

## Description of the Drawings

Further understanding of the invention will be facilitated by reference to the following drawings wherein like reference numerals refer to like parts, and

FIGURE 1 is a perspective view of the bioelectrode of the invention, somewhat out of proportion, having parts thereof peeled back and other parts broken;

FIGURE 2 is a greatly enlarged sectional view of the electrode of Figure 1, showing the attachment of the lead wire to the electrode; and

FIGURE 3 is an alternative embodiment of the bioelectrode of the invention.

## Detailed Description

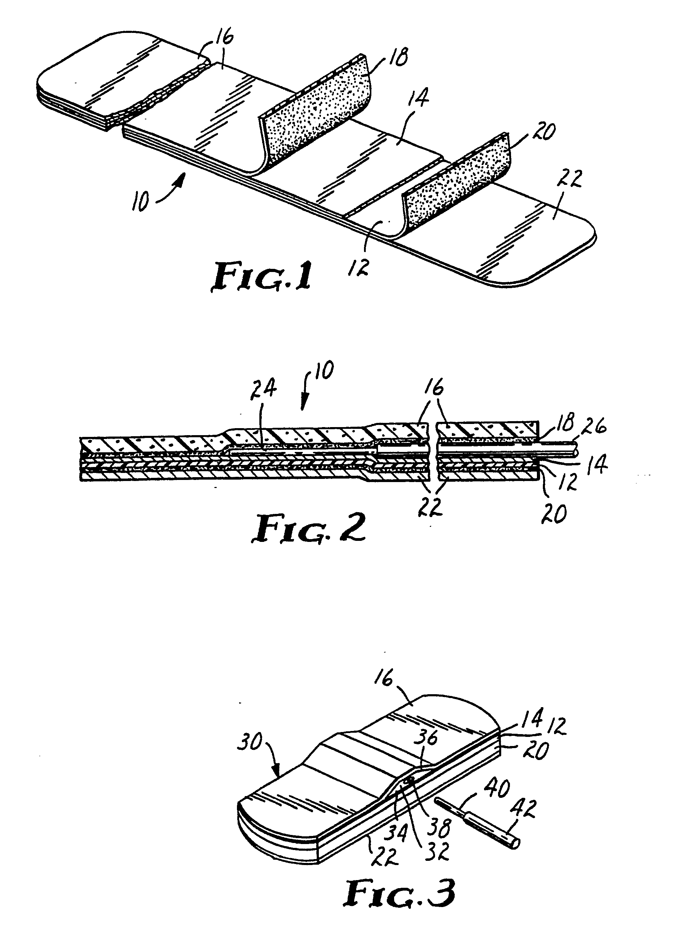

Referring now to FIGURES 1 and 2, biomedical electrode 10 is primarily designed as a post-operative TENS electrode. It is elongated and generally rectangular in shape. Electrode 10 comprises a flexible, non-metallic electrically-conductive film 12. Film 12 conducts current electronically, not ionically, and is, therefore, a barrier to the migration of metallic ions. Film 12 is preferably formed from a thermoplastic resin which has been made electrically conductive by the inclusion of conductive carbon particles such as carbon black. A layer of conductive metal 14 overlies the top surface of film 12 and is in good electrical contact therewith over substantially all of its surface area on the side contacting film 12. Preferably, metal layer 14 is a thin layer of silver vapor deposited onto the top surface of film 12.

An extensible, electrically-insulating backing 16 overlies the side of metal layer 14 opposite to that contacting film 12, and is adhered to metal layer 14 by means of adhesive 18. Backing 16 adds to the mechanical integrity of the electrode and protects the fragile metal layer 14 from damage. Backing 16 must be extensible enough to stretch in response to the mechanical forces to which the electrode is exposed during application to the body and subsequent use without stressing the metal layer 14 and causing it to delaminate from and lose contact with film 12. Backing 16 must also be comfortable to body contours so as not to detract from the overall suppleness of the electrode. The particularly preferred material for backing 16 is PVC foam tape, e.g., Microfoam (Registered Trade Mark) Surgical Tape sold by the 3M Company, St. Paul, Minnesota U.S.A. This tape is one of the most conformable and extensible tapes available. Also suitable is an adhesive film sold under the registered trademark Tegaderm by the 3M Company for use as a wound dressing. This is polyurethane film coated on one side with a pressure sensitive adhesive.

This material is thinner than Microfoam Surgical Tape and may be preferred for elelectrodes such as that illustrated in FIGURE 3 which is slightly thicker than the electrode of FIGURES 1 and 2 to minimize overall thickness. Conformable, electrically conductive interfacing material 20 is coated onto the bottom surface of film 12. Interfacing material 20 can be any gel, paste or conductive adhesive conventionally used in biomedical electrodes to reduce impedance and enhance the electrical connection between the electrode and the skin. For ease in manufacture and handling, so called "dry" conductive adhesives such as those described in U.S. Patent No. 4,066,078; U.S. Patent No. 4,352,359; and International Application No. PCT/US80/01543 are preferred. Especially preferred is the class of adhesive described in the aforementioned U.S. Patent No. 4,352,359 such as a copolymer of n-butyl acrylate and acrylic acid (75:25) neutralized with methyl diethanolamine. These adhesives are sufficiently tacky to hold the electrode securely on the skin, and because of their low water content, do not dry out during use. Interfacing material 20 may vary greatly in thickness between about (0.05 mm—1.78mm) (2 and 70 mils) depending on the type of interfacing material and the intended use of the electrode. In the embodiment illustrated in FIGURE 1, designed for use as a disposable post-operative TENS electrode, interfacing material 12 is preferably about 0-1 mm (4 mils) thick.

Interfacing material 20 is protected prior to use by a conventional release liner 22.

Electrode 10 is connected to an electromedical device such as a stimulator by way of wire 24, illustrated in FIGURE 2. Wire 24 is encased in insulating sheath 26 and sandwiched between backing 18 and metal layer 14. A length approximately 19 mm (3/4 inch) long of wire 24 is exposed from the end of sheath 26 and in intimate electrical contact with metal layer 14. Metal layer 14 distributes the current evenly throughout its surface area where it is conducted via film 12 and interfacing material 20 to the skin underlying the electrode.

The embodiment of FIGURE 3 is an alternative embodiment of the bioelectrode of the invention. The electrode 30, as illustrated is rectangular in shape, but any desired shape may be used. Electrode 30 is smaller in length and width than the embodiment shown in FIGURE 1 and may be used for any type of application where electrical current is delivered to the body such as TENS, F.E.S. or, possibly, iontophoretic drug delivery. Most of the elements of the electrode are similar to those of the electrode of FIGURES 1 and 2, except the layer of interfacing material 20 is thicker (and may be drug containing gel or paste), the backing 16 is thinner, and the means for connection to the stimulator is different.

Electrode 30 contains a body-conformable one-piece electrically-conductive connector 32 having a substantially flat lower surface 34 overlying and in electrical contact with a portion of metal layer 14. Backing 16 overlies the top surface 36 of connector 34 and the portion of metal layer 14 not covered by connector 34, and is adhered thereto by a thin layer of adhesive (not shown). Connector 34 has a passageway 38 extending through the central portion thereof with an opening on each lateral face of the electrode. Passageway 38 serves as a female receptacle for plug 40 which is connected to the lead wire 42 of an electromedical stimulator.

Connector 34 distributes current to the underlying metal layer 14 and is preferably made of carbon-impregnated silicone rubber (e.g., Silicone Rubber Compound C-968, SWS Silicones Corporation, Adrian, Michigan 49221). Other suitable carbon impregnated polymeric materials include plasticized polyvinyl chloride, epichlorohydric rubber, polyurethane molding compound, polytetrafluoro-ethylene and a polymer of ethylene-propylene-diene (e.g., EPDM rubber).

The interfacing material 20 of electrode 30 is slightly thicker than that shown in the embodiment of FIGURES 1 and 2, e.g., on the order of 10-70 mils (0.0254-0.1778 cm). If used for iontophoretic drug delivery, the drug may be incorporated into the interfacing layer and the interfacing layer tends to be thicker.

The essence of the present invention lies in the unique combination of metal layer 14 with film 12 which serves as a barrier against the migration of metal ions from metal layer 14 to the skin. The preferred composition of film 12 is 71 per cent ethylene vinyl acetate (Grade UE 632 "Ultrathene" from USI Chemicals) and 29 percent carbon black (X—C 72, Cabot Corporation). The ingredients are mixed, pellitized and extruded using conventional' techniques to form a film, having a thickness in the general range of 2 to 4 mils (50 to 100 micrometers). The preferred thickness is about 3 mils (approximately 75 micrometers).

Although ethylene vinyl acetate is the presently preferred thermoplastic resin for use in forming film 12, a variety of other resins, such as PVC, polyethylene and polypropylene, can be used to achieve the requisite conformability and conductivity when mixed with electrically conductive carbon. Carbon black is the preferred conductive carbon, and it typically comprises between about 25 and 30 per cent of the film. Below a concentration of about 25 per cent, conductivity falls off, and minimal advantages are perceived by adding more than 30 per cent.

Metal layer 14 conducts the currently evenly over the entire area of the electrode. Metal layer 14 is preferably vapor deposited on one surface of film 12. Silver is the present metal of choice, and a layer having a thickness of about 40 nm (about 300 mcg/sq.[2]) is preferred and easily applied using conventional techniques for vapor deposition of metals. Vapor coating is economical and provides better electrical contact with film 12 and better suppleness than is obtainable with metal foil or meshes.

In addition to providing a barrier to the migration of metal ions from metal layer 14 to the skin, film 12 has the additional advantage that its electrical conductivity is relatively unaffected by flexing and bending. A woven material, on the other hand, is difficult to coat evenly with metal, and, when flexed, movement of fibers relative to each other interferes with electrical conductivity.

To use the biomedical electrode of the present invention as illustrated in FIGURES 1-3, the release liner 22 is removed, and the electrode is placed on the desired surface of the skin. Prior to placing the electrode on the skin, the lower surface of interfacing material 20 or the skin may be wetted with water to optimize adherence and conductivity of the skin adhesive interphase. The electrode is then connected to an electromedical device.

Electrodes of the present invention have been found to have a very low impedance, on the order of 30 ohms at 60-80 Hz.

To determine direct current stability, electrodes according to the present invention were placed on an agar surface (agar 1.5%, KCl 8.0%, water 90.5%). Direct current (1 microamp) was passed through the electrodes, used as both the anode and cathode, for 24 hours. No physical change was observed in any of the electrodes. No deplating of the metal layer was detected, and the electrodes were able to function normally after the test.

**Claims**

1. A biomedical electrode (10) comprising an electrically conductive metallic member (14), means (24) for electrically connecting said metallic member (14) to an electromedical device, a flexible, electrically-conductive, non-metallic film (12) which is a barrier to the migration of metallic ions, said non-metallic film (12) having a top surface and a bottom surface, and an extensible, electrically insulating backing (16) which overlies and is adhered to said metallic member (14) on the side opposite to the side contacting said film (12); characterised in that said electrically-conductive metallic member (14) is in the form of a layer (14) which overlies and is in electrical contact with substantially all of said top surface of said film (12), thereby conducting current evenly over the entire area of the electrode.

2. An electrode according to claim 1 further characterized by a coating of a comformable, electrically-conductive interfacing material (20) on said bottom surface of said film (12).

3. An electrode according to claim 2 further characterized in that said interfacing material (20) is an electrically-conductive pressure sensitive adhesive.

4. An electrode according to claim 3 further characterized in that said electrically-conductive pressure-sensitive adhesive is a copolymer of n-butyl acrylate and acrylic acid neutralized with methyl diethanolamine.

5. An electrode according to claim 3 or 4 further characterized by a release liner (22) adhered to the exposed surface of said coating of pressure-sensitive adhesive (20).

6. An electrode according to claim 2, 3, 4 or 5 further characterized by a drug contained in said interfacing material capable of iontophoretic delivery to the skin.

7. An electrode according to any preceding claim further characterized in that said film (12) comprises a thermoplastice resin containing an effective amount of electrically-conductive carbon to render said resin electrically-conductive.

8. An electrode according to claim 7 further characterized in that said resin is ethylene vinyl acetate.

9. An electrode according to claim 7 or 8 further characterized in that said electrically-conductive carbon is carbon black.

10. An electrode according to claim 9 further characterized in that said carbon black comprises between about 25 and 30 per cent of said film (12).

11. An electrode according to any preceding claim further characterized in that said metallic layer (14) comprises silver.

12. An electrode according to claim 11 further characterized in that said silver is vapor-deposited on said top surface of said film (12).

13. An electrode according to any preceding claim further characterized in that said backing (16) comprises a polyvinyl chloride foam tape.

14. An electrode according to any one of claims 1-12 further characterized in that said backing (16) comprises a thermoplastic polyurethane film having a layer of pressure sensitive adhesive on the side thereof contacting said metallic layer (14).

15. An electrode according to any preceding claim further characterized in that said means (24) for electrically connecting said metallic layer (14) to an electromedical device is an electrically-conductive wire (24) sandwiched between said metallic layer (14) and said backing (16) in electrical contact with said metallic layer (14).

16. An electrode according to any one of claims 1-14 further characterized in that said means (24) for electrically connecting said metallic layer (14) to an electromedical device is a one-piece electrically-conductive connector (32) sandwiched between said metallic layer (14) and said backing (16) with at least one end thereof exposed, said connector (32) having a substantially flat lower surface (34) overlying a portion of said metallic layer (14) and in electrical contact therewith and a female receptacle (38) in said exposed end for receiving an electrical plug (40) for attachment to said electromedical device.

**Patentansprüche**

1. Biomedizinische Elektrode (10) mit einem elektrisch leitenden, metallischen Glied (14), Mitteln (24) zum elektrischen Verbinden des metallischen Gliedes (14) mit einer elektromedizinischen Vorrichtung, einem flexiblen, elektrisch leitenden nicht metallischen Film (12), der eine die Wanderung von Metallionen verbindende Sperre bildet und eine obere und eine untere Fläche hat, und einer dehnbaren, elektrisch isolierenden Tragschicht (16), die über dem metallischen Glied (14) liegt und mit ihm auf der Seite verklebt ist, die der mit dem Film (12) in Berührung stehenden Seite entgegengesetzt ist, dadurch gekennzeichnet, daß das elektrisch leitende metallische Glied (14) aus einer Schicht (14) besteht, die über der genannten oberen Fläche des Films (12) liegt und mit ihr im wesentlichen in ihrer ganzen Ausdehnung in Berührung steht, so daß sie Strom in gleichmäßiger Verteilung über der ganzen Fläche der Elektrode leitet.

2. Elektrode nach Anspruch 1, gekennzeichnet durch einen Überzug aus einem auf der unteren Fläche des Films (12) vorgesehenen und ihr angepaßten Überzug aus einem elektrisch leitenden Zwischenschichtmaterial (20).

3. Elektrode nach Anspruch 2, dadurch gekennzeichnet, daß das Zwischenschichtmaterial (20) ewin elektrisch leitender Haftkleber ist.

4. Elektrode nach Anspruch 3, dadurch gekennzeichnet, daß der elektrisch leitende Haftkleber ein mit Methyldiethanolamin neutralisiertes Copolymer des n-Butylacrylats und der Acrylsäure ist.

5. Elektrode nach Anspruch 3 oder 4, gekennzeichnet durch eine mit der freiliegenden Fläche des Überzuges aus dem Haftkleber (20) verklebte Antihaftauflage (22).

6. Elektrode nach Anspruch 2, 3, 4 oder 5, dadurch gekennzeichnet, daß das Zwischenschichtmaterial ein zur iontophoretischen Abgabe an die Haut geeignetes Medikament enthält.

7. Elektrode nach einem der vorhergehenden Ansprüche, dadurch gekennzeichnet, daß der Film (12) mindestens teilweise aus einem thermoplastischen Harz besteht, das elektrisch leitfähigen Kohlenstoff in einer solchen Menge enthält, daß das Harz elektrisch leitfähig ist.

8. Elektrode nach Anspruch 7, dadurch gekennzeichnet, daß das Harz Ethylenvinylacetat ist.

9. Elektrode nach Anspruch 7 oder 8, dadurch gekennzeichnet, daß der elektrisch leitfähige Kohlenstoff Ruß ist.

10. Elektrode nach Anspruch 9, dadurch gekennzeichnet, daß der Anteil des Rußes in dem Film (12) etwa 25 bis 30 Prozent beträgt.

11. Elektrode nach einem der vorhergehenden Ansprüche, dadurch gekennzeichnet, daß die Metallschicht (14) mindestens teilweise aus Silber besteht.

12. Elektrode nach Anspruch 11, dadurch gekennzeichnet, daß das Silber auf der oberen Fläche des Films (12) aufgedampft ist.

13. Elektrode nach einem der vorhergehenden Ansprüche, dadurch gekennzeichnet, daß die Tragschicht (16) mindestens teilweise aus einem Band aus Polyvinylchloridschaumstoff besteht.

14. Elektrode nach einem der Ansprüche 1 bis 12, dadurch gekennzeichnet, daß die Tragschicht (16) mindestens teilweise aus einem thermoplastischen Polyurethanfilm besteht, der auf der mit der metallischen Schicht (14) in Berührung stehenden Seite eine Schicht aus einem Haftkleber besitzt.

15. Elektrode nach einem der vorhergehenden Ansprüche, dadurch gekennzeichnet, daß die Mittel (24) zum elektrischen Verbinden der Metallschicht (14) mit einer elektromedizinischen Einrichtung aus einem elektrisch leitenden Draht (24) bestehen, der zwischen der metallischen Schicht (14) und der Tragschicht (16) angeordnet ist und mit der metallischen Schicht (14) elektrisch in Kontakt steht.

16. Elektrode nach einem der Ansprüche 1 bis 14, dadurch gekennzeichnet, daß die Mittel (24) zum elektrischen Verbinden der metallischen Schicht (14) mit einer elektromedizinischen Einrichtung aus einem einteiligen, elektrisch leitenden Verbinder (32) bestehen, der zwischen der metallischen Schicht (14) und der Tragschicht (16) angeordnet ist und der mindestens an seinem einen Ende freiliegt und der eine im wesentlichen ebene untere Fläche (34) besitzt, die über einem Teil der Metallschicht (14) liegt und mit ihr in elektrischem Kontakt steht, sowie aus einer in dem freiliegenden Ende vorgesehenen Aufnahme (38) für einen elektrischen Stecker (40) zum Anbringen an der elektromedizinischen Einrichtung.

**Revendications**

1. Electrode biomédicale (10) comprenant un élément métallique électriquement conducteur (14), des moyens (24) pour la liaison électrique duduit élément métallique (14) à un dispositif électromédical, un film non métallique (12), flexible, électriquement conducteur, qui constitue une barrière à la migration des ions métalliques, ledit film non métallique (12) présentant une surface supérieure et une surface inférieure, et un support extensible électriquement isolant (16) qui recouvre et est collé audit élément métallique (14) sur la face opposée à la face en contact avec ledit film (12), caractérisée en ce que ledit élément métallique électriquement conducteur (14) est sous la forme d'une couche (14) qui recouvre et est en contact électrique avec sensiblement toute ladite surface supérieure dudit film (12), de manière à conduire le courant uniformément sur toute la surface de l'électrode.

2. Electrode suivant la revendication 1, caractérisée en ce qu'elle comprend, en outre, un revêtement d'une matière d'interface conformable, électriquement conductrice (20), sur ladite surface inférieure dudit film (12).

3. Electrode suivant la revendication 2, caractérisée en outre en ce que ladite matière d'interface (20) est un adhésif électriquement conducteur, sensible à la pression.

4. Electrode suivant la revendication 3, caracérisé en outre en ce que ledit adhésif électriquement conducteur sensible à la pression est un copolymère de n-butylacrylate et d'acide acrylique neutralisé avec la méthyl diéthanolamine.

5. Electrode suivant la revendication 3 ou 4, caractérisée en ce qu'elle comprend, en outre, une doublure détachable (22), collée à la surface exposée dudit revêtement d'adhésif sensible à la pression (20).

6. Electrode suivant la revendication 2, 3, 4 ou 5, caractérisée en outre par un médicament contenu dans ladite matière d'interface et qui peut être administré par ionophorèse à la peau.

7. Electrode suivant l'une quelconque des revendications précédentes, caractérisée en outre en ce que ledit film (12) comprend une résine thermoplastique contenant une quantité efficace de carbone électriquement conducteur, pour rendre ladite résine électriquement conductrice.

8. Electrode suivant la revendication 7, caractérisée en outre en ce que ladite résine est de l'acétate de vinyl-éthylène.

9. Electrode suivant la revnedication 7 ou 8, caractérisée en outre en ce que ledit carbone électriquement conducteur est du noir de carbone.

10. Electrode suivant la revendication 9, caractérisé en outre en ce que ledit noir de carbone constitue entre 25 et 30% environ dudit film (12).

11. Electrode suivant l'une quelconque des revendication précédentes, caractérisée en outre en ce que ladite couche métallique (14) comprend de l'argent.

12. Electrode suivant la revendication 11, caractérisé en outre en ce que ledit argent est déposé par vapeur sur ladite surface supérieure dudit film (12).

13. Electrode suivant l'une quelconque des revendications précédentes, caractérisé en outre en ce que ledit support (16) est un ruban de mousse de chlorure de polyvinyle.

14. Electrode suivant l'une quelconque des revendications 1 à 12, caractérisée en outre en ce que ledit support (16) est un film de polyuréthane thermoplastique comportant une couche d'adhésif sensible à la pression, sur sa face en contact avec ladite couche métallique (14).

15. Electrode suivant l'une quelconque des revendications précédentes, caractérisée en outre en ce que lesdits moyens (24) pour la liaison électrique de ladite couche métallique (14) à un dispositif électromédical comprennent un fil électriquement conducteur (24) interposé entre ladite couche métallique (14) et ledit support (16), en contact électrique avec ladite couche métallique (14).

16. Electrode suivant l'une quelconque des revendications 1 à 14, caractérisée en outre en ce que lesdits moyens (24) pour la liaison électrique de ladite couche métallique (14) à un dispositif électromédical comprennent un connecteur monobloc électriquement conducteur (32), interposé entre ladite couche métallique (14) et ledit support (16), au moins une extrémité de ce connecteur étant exposée, ledit connecteur (32) présentant une surface inférieure sensiblement plane (34), qui est superposée à une partie de ladite couche métallique (14) et en contact électrique avec celle-ci, et un réceptacle femelle (38) dans ladite extrémité exposée, pour recevoir une broche électrique (40) de raccordement audit dispositif électromédical.

FIG.1

FIG. 2

FIG.3

1